# EUROPEAN PATENT APPLICATION

(11) **EP 1 067 466 A2**
(43) Date of publication of application: **10.01.2001**
(21) Application number: 00202402.4
(22) Date of filing: 06.07.2000
(51) Int. Cl.: G06F 17/30, G06F 19/00

(54) **Genome browser interface**

(30) Priority: 09.07.1999 US 143109 P
(71) Applicant: Smithkline Beecham, Philadelphia, PA 19103 (US)
(72) Inventor: Delaney, Shawn, c/o SmithKline Beecham Corporation, Philadelphia, PA 19103 (US); Hudson, Douglas, c/o SmithKline Beecham Corp., Philadelphia, PA 19103 (US); Miller, Jason, c/o SmithKline Beecham Corporation, Philadelphia, PA 19103 (US); Ong, Irene, c/o SmithKline Beecham Corporation, Philadelphia, PA 19103 (US); Swift, Roderick, c/o SmithKline Beecham Corp., Philadelphia, PA 19103 (US); van der List, Michael, c/o SmithKline Beecham Corp, Philadelphia, PA 19103 (US)
(74) Representative: Blakey, Alison Jane

(57) **Abstract**

A genome browser comprising a spreadsheet interface to organize annotation.

## Description

### Scope of the Invention

Genome browsers are software tools for visualizing a DNA sequence in the context of its annotation. Genome browsers are critical tools for assisting the analytical task of manually integrating annotations from various algorithms, none of which are 100% trusted. All genome browsers use some combination of stacking and banding annotations around a sequence. The spreadsheet is a vehicle for presenting stacked and banded annotation. Hewewith is presented a new genome browser that uses a spreadsheet-like interface to organize annotation. Its layout is useful and simple to implement using pre-existing software libraries.

### Background

Many genome browsers exist. These browsers are software tools for visualizing recorded human interpretations of DNA sequences. Browsers typically present sequence and annotation together at some user-settable scale. Some genome browsers support the creation and storage of "manual annotations" derived during the visual experience.

The need for manual annotation may result from the inaccuracy of computational analysis. For instance, many computational gene finding methods exist, but each method is far from perfect (Burset & Guigó, 1996). The computational combination of methods, for instance by machine learning, is also imperfect. In this environment, the best gene finding method may be case-by-case analysis by expert human annotators who integrate and evaluate computational predictions.

Presumably, browsers assist manual annotation by visually integrating diverse data. All browsers present sequence and annotation in close proximity. Typically, the sequence is represented in two-dimensional space as a line, possibly wrapping like text, with annotations denoted by colored, labeled shapes packed along either side of the sequence. Visual proximity of sequence to annotation is apparently a goal of browser cartography. All known browsers accomplish this goal by combining two layout techniques.

The first common layout uses stacking. Every annotation is drawn as close to the sequence as possible without overlapping previously drawn annotations. In sparsely annotated regions, every annotation abuts the sequence, but elsewhere the annotations stack up. In this layout, an annotations' distance from the sequence conveys no information. ACeDB (Thierry-Mieg & Durbin 1999) displays mapped clones using the stacked layout. The bioTk tools (Searls, 1995) and derivative browsers also stack annotations, as do the browsers ChromeView (BDGC, 1999), AnnotationViewer (Crabtree *et al*., 1998), the KEGG browser (Ogata, 1999), and the BioViews tools (Helt *et al*., 1998).

The second common layout introduces bands. Annotations appear in bands parallel to the sequence, with each band restricted to an analytical category. Although the bands are stacked close to the sequence, individual annotations may not be, especially in sparsely annotated regions. The banding layout sacrifices some proximity for the clarity of separation. The banded layout is popular for showing gene predictions. The Genome Channel browser (Mural, 1999) groups annotations into horizontal bands labeled "Grail exons," "Genscan exons," etc. Each band occurs at a fixed distance from the sequence. Each band occurs above and below the sequence to further distinguish annotations by DNA strand. Other browsers that employ similar layouts include Genotator (Harris, 1997), GeneScope (Murakami & Takagi, 1998), and HAGIS (Taylor *et al*., 1998). CloneView (BDGC, 1999) uses two levels of banding. Each of two windows has bands within, but the windows themselves are tiled and serve to distinguish computational vs. manual annotation. The commercial product Lasergene also uses banding in some modules, such as Protean (DNAStar, 1998).

The banded layout has the advantage of segregating data by category. The stacked layout has the advantage of maximal proximity. Most browsers, including those referenced so far, implicitly combine both alternatives. Some browsers make the combination explicit. DerBrowser (Grigoriev, 1996) displays stacks of annotations subtly organized into "stripes." DerBrowser's menu lets users toggle the display of each stripe. Genotator (Harris, 1997) uses banding explicitly while some bands, such as ORF bands, contain stacking annotations.

### Summary of the Invention

In a first aspect this invention relates to an improved genome browser wherein the improvement comprises using a tabular layout for annotating genomic.

In a second aspect genomic browser named comprising a Java application that presents DNA sequence annotations inside a spreadsheet.

### Description of the Figures

Figure 1 is a screenshot of the browser.
Figure 2 is sample user configuration file.

### Detailed Description of the Invention

We provide a genome browser interface that maximizes the combined benefits of the stacked and banded layouts. Its tabular layout can accommodate the need for simultaneous stacking and banding in genomic annotation. Herein we describe a new genomic browser named "Gadget" (for genomic annotation widget), a Java application that presents DNA sequence annotation inside a spreadsheet. Gadget's columns contain either textual detail or represent a span of sequence. Gadget's rows each contain some user-definable category of annotation. Some cells in Gadget's table contain program objects that render physical maps, all of which scroll and zoom in unison.

Gadget's interface lends analytic power to users familiar with its data manipulation techniques. The spreadsheet interface is familiar to most computer users. Gadget users drag-specific rows of annotations close to the sequence and thereby accomplish the visual proximity goal for which all genome browsers strive.

Gadget reads a configuration file at startup. The configuration file determines the label, order, and contents of every row in Gadget's main table. The configuration file may assign annotations to rows according to these data attributes: source, feature, strand, frame. For convenience, Gadget generates a default configuration file on first use. Users can edit their configurations with any text editor.

Gadget reads and writes data from flat files in the GFF format (Durbin & Haussler, 1999). The GFF specification permits unspecified name/value pairs in the comments column. Gadget overloads the comments column to save information such as the modification date and username of every edited annotation.

Gadget was written in Java and deployed as a Unix application. As initially deployed, Gadget was compiled with the Java Development Kits (JDK) 1.1 from Sun Microsystems (http://www.sun.com/) and Digital Equipment Corporation (http://www.dec.com/). Gadget used the software libraries NGSDK 1.0.4 by Neomorphic Software (http://www.neomorphic.com) and JClass 3.6.0 by KL Group (http://www.klg.com). Gadget was later enhanced using Sun JDK 1.2. including Swing 1.1, NGSDK 1.1.1, and JClass 3.6.1.

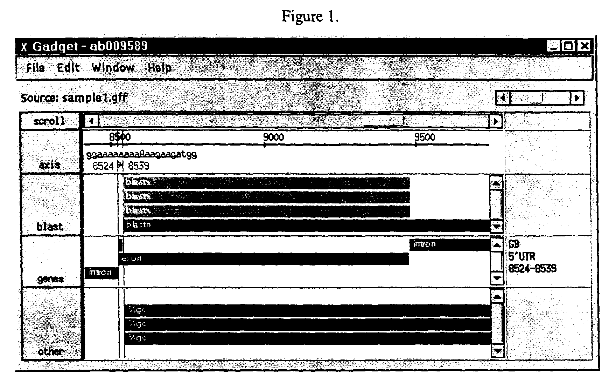

Gadget's tabular displays were implemented with the JCTable widget from the JClass library. The equivalent table widget in the Swing library was rejected due to lack of sufficient row-wise support. Java pop-up menus (from the Swing and AWT libraries) were used in place of the JCTable' s built-in pop-up menus due to many final attributes declared by the latter. Gadget's physical maps and editable sequence were implemented with the NeoMap widget from the NGSDK library. Gadget's zoom capability relied on the NeoMap's semantic zoom feature. Gadget's wrapped sequence display was built from AWT components. Gadget's drawing objects were implemented as custom subclasses of the NGSDK's basic drawing class, called Glyph. These objects include all the annotation drawings plus the lens, extents, and editable sequence (see figure 1).

Gadget's spreadsheet has two columns. Figure 1 is a screenshot of Gadget's main menu. The left column contains row labels and textual details. The right column contains graphic genomic maps. The rows and columns can be interchanged, using drag and drop. In this configuration Gadget is displaying four rows and three columns. Column 1 labels each row. Column 2 contains the graphic genome maps. Column 3 contains details about the annotation under the mouse pointer. In this screenshot, the mouse pointer was over the green 5'untranslated region (UTR) annotation in the 3^{rd} row, labeled "genes". Row 1, labeled "axis," contains the sequence axis (black), the sequence lens (green and black text) which magnifies the 21 bases around the mouse pointer, and the annotation extents (red vertical lines and base indices) which delineate the full extent of the annotation under the mouse pointer. The other rows contain three categories of annotation, "blast", "genes", and "other". Users can configure the number of rows, the labels on each row, and the category of annotation that each row contains. Users also configure the color and label on each annotation.

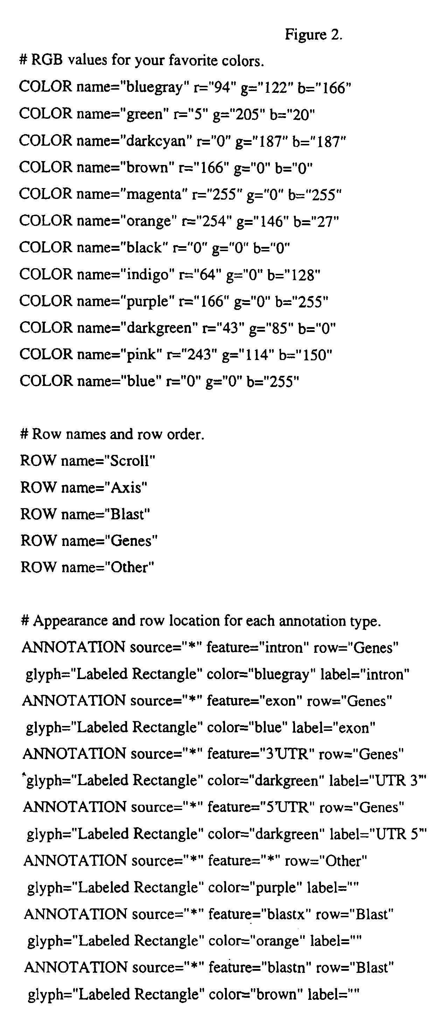

Gadget's initial layout depends on a configuration file read once at startup. Figure 2 is a sample user configuration file for Gadget. The configuration specifies, for example, that exon, intron, and UTR annotations shall display in the penultimate row, which shall be labeled "Genes." The configuration shown was used to generate the screenshot set out in figure 1.. The configuration file specifies the row labels, the row order, and the row contents. For example, one configuration might load Genie predictions in one row and Genscan predictions in another. Different configuration files can generate a wide variety of initial layouts.

Users can change gadget's display interactively. Users can move certain rows closer to the sequence than others, using drag and drop. Users can increase the space allocation for some rows by collapsing or deleting other rows, by dragging borders with their mouse. Users can organize annotations subjectively by moving arbitrary collections of annotations from one row to another, using select, cut, and paste. Users can create annotations, or edit existing ones, by activating the annotation property edit window.

Gadget's textual details column is responsive to the user's mouse movements. When no annotations are selected, Gadget's text always describes the annotation under the mouse, if any. When one annotation is selected (per row), the text for that row describes that annotation. Thus, users can compare the textual details of two annotations in separate columns by selecting each. Unfortunately, users cannot do this for two annotations in the same column.

Like most browsers, Gadget represents annotations with labeled, colored shapes. Gadget represents most annotations as block arrows whose direction indicates strand. It represents the exons with a gene model as colored arrows within a larger, gray rectangle. It displays alignments as single lines of text, where unaligned inserts are condensed until a mouse operation provokes their expansion. Gadget's color choices are user-settable via the configuration file.

Gadget's graphic maps are responsive to mouse movements. In the axis row, an optional "lens" feature displays the 21 -bp window of nucleotides immediately surrounding the mouse pointer. The nucleotides are colored to highlight the exact boundary of the annotation under the mouse, if any. Another optional feature displays "extents," vertical lines across all rows highlighting the boundaries of the annotation under the mouse, if any.

Gadget's scroll and zoom controls effect all of Gadget's rows in unison. At minimum zoom, the entire genomic sequence is in view. At maximum zoom individual nucleotides are visible over a short range of sequence. Gadget zooms "towards" or "away from" the selected annotation, if any. Every annotation's drawing carries a label that displays only when the zoom scale permits so "closer" zoom affords greater detail. Gadget's zoom can be adjusted at very fine grain with acceptable performance.

Gadget is unique amongst genomic sequence browser because it offers an explicit spreadsheet interface. The spreadsheet layout was implemented from pre-existing software components. The spreadsheet is an effective presentation technique for genome browsing and editing. Gadget's interface is immediately useable by untrained users. The spreadsheet layout offers a manual solution to one problem common to all genome browsers: with two-dimensional non-overlapping representations, not all annotations can be proximal to the sequence. Thus, Gadget lets users control its most critical layout decisions, the grouping and proximity of annotations and sequence.

Gadget lets users visually organize annotations to assist their analysis. The labeled rows and columns of Gadget's spreadsheet immediately convey relationships among the data. Gadget exploits the spreadsheet metaphor by suggesting that users move, collapse, or remove whole rows of annotations. Gadget extends the spreadsheet metaphor by including a graphic map within each data row.

The use of a configuration file gives users powerful control over Gadget's startup display. Gadget offers menu support for using multiple, saved configurations.

In one embodiment, Gadget's spreadsheet is limited to exactly two columns. Other embodiments dispense with Gadget's column number limitation. Multiple columns could display different genes, or distant portions of the same genome, or syntenic regions of different genomes, all in the same spreadsheet. Yet another embodiment of Gadget could support collapsing portions of a genome thus bringing together, say, distant exonic sequences. "Hide column" is a feature well known to spreadsheet users.

### References

Benson D.A., Boguski M.S., Lipman D.J., Ostell J., Ouellette B.F.F., Rapp B.A., Wheeler D.L. (1999) GenBank. *Nucleic Acids Research*, **27**(1): 12-17.
Berkeley Drosophila Genome Center (1999) ChromoView v. 2.0 Launch Page. http://weasel.lbl.gov/displays/chromoview.html.
Burset M., Guigó R. (1996) Evaluation of gene structure prediction programs. *Genomics* **34**: 353-357.
Crabtree J., Fischer S., Gibson M. (1998) CBIL bioWidgets Demo: AnnotView. http://www.cbil.upenn.edu/bioWidgets/annotViewDemo/index.html.
DNAStar (1998) Protein Structure Discovery, Annotation and Publication. http://www.dnastar.com/products/Protean.html.
Durbin R., Haussler D. (1999) GFF (Gene Finding Features) Specifications Document. http://www.sanger.ac.uk/Software/GFF/GFF_Spec.shtml.
Grigonev A. (1996) DerBrowser: Genome Navigator. http://www.mpimg-berlin-dahlem.mpg.de/~andy/DerBrowser.
Harris N.L. (1997) Genotator: A workbench for sequence annotation. *Genome Research* **7**(7): 754-762.
Helt G.A., Lewis S., Loraine A,E., Rubin G.M. BioViews - Java-based tools for genomic data visualization. PCR *Methods & Applications*, **8**(3): 291-305.
Murakami K., Takagi T. (1998) Gene recognition by combination of several gene-finding programs. *Bioinformatics*, **14**(8): 665-675.
Mural R.J., Parang M., Shah M., Snoddy J., Uberbacher E.C. (1999) The Genome Channel: a browser to a uniform first-pass annotation of genomic DNA. *Trends in Genetics*, **15**(1): 38-39.
Ogata H., Goto S., Sato K., Fujibuchi W., Bono H., Kanehisa M. (1999) KEGG: Kyoto encyclopedia of genes and genomes. *Nucleic Acids Research*, **27**(1): 29-34.
Searls D.B. (1995) bioTk: componentry for genome informatics graphical user interfaces. *Gene*, **163**: GC1-GC16.
Thierry-Mieg J., Durbin R. (1999) ACEDB. http://www.sanger.ac.uk/Software/Acedb/.

## Claims

1. A genome browser comprising a spreadsheet interface to organize annotation.

2. The browser of claim 1 wherein the interface comprises a Java application presents DNA sequence annotations inside said spreadsheet.

3. A method of organizing annotation in a genome browser comprising using a spreadsheet interface.

4. An improved genome browser wherein the improvement comprises using a tabular layout for annotating genomic.
